# EUROPEAN PATENT APPLICATION

(11) **EP 1 304 139 A1**
(43) Date of publication of application: **23.04.2003**
(21) Application number: 00981970.7
(22) Date of filing: 30.11.2000
(51) Int. Cl.: A61N 5/00

(54) **DEVICE FOR POLYFACTORIAL CURING**

(30) Priority: 17.07.2000 RU 2000119051
(71) Applicant: Datchenko, Alexandr Andreevich, Taganrog 347900 (RU); Mnushko, Vladimir Nikolaevich, Taganrog 347900 (RU)
(72) Inventor: Datchenko, Alexandr Andreevich, Taganrog 347900 (RU); Mnushko, Vladimir Nikolaevich, Taganrog 347900 (RU)
(74) Representative: Beetz & Partner Patentanwälte
(86) International application number: RU0000490
(87) International publication number: WO02005894

(57) **Abstract**

The invention relates to medicine, in particular to physiotherapy. The device may be used for preventing and curing diseases of the central nervous system, the vegetative nervous system, the respiratory organs, the digestive organs, of metabolic disturbances and involutional changes, the diseases of the cardiovascular system, of the supporting apparatus and the motor system. The device comprises two layers (2, 3) of woven, knitted or needle-punched fabric, between which a screen (4) with a high reflection factor for the electromagnetic waves of the ultrahigh frequency (UHF) and the infra-red spectrum is arranged. The screen is made of a synthetic film (5) having a metallization layer (6) onto which a layer of a polymeric material (7) is applied. The technical result provides an increase of the curing influence.

## Description

The invention relates to medicine, more particularly to the field of physiotherapy. The device can be used for preventing and curing diseases of the central nervous system, the vegetative nervous system, the respiratory organs, the digestive organs, metabolic disturbances and involutional changes, the diseases of the cardiovascular system, the supporting apparatus and the motor system.

The closest device as compared with the proposed device is a blanket for polyfactorial curing which contains at least three layers, with at least two layers forming the electrically insulating material, a screening layer of synthetic films being arranged between the electrically insulating layers, which is provided with a metallization and having a high reflection factor for the electromagnetic waves of the ultrahigh frequency (UHF) and the infra-red spectrum.

According to the description, the blanket promotes multilateral recovery of the organism and is used for preventing and curing various diseases of the nervous system, the supporting apparatus and the motor system, as well as cardiovascular diseases.

When using the prototype, the curing effect is achieved in that a local milieu is created by enveloping parts of the body or the whole body with the blanket, which is characterized by the following power factors:
- full or partial protection of the patient against the influence of external electrostatic and electromagnetic fields;
- influence on the patient by his own reflected electromagnetic radiation of the ultrahigh frequency (UHF);
- influence on the patient by his own reflected electromagnetic radiation of the infra-red range;
- redistribution of the free electric charge on the skin surface of the patient.

The deficiency of the prototype is the gradual decrease of the curing effect during the realization of the procedure. The decrease of the curing effect is explained by a comparable fast leakage of the free electric charge from the skin surface of the patient's body to the screening layer during the procedure. The reduction of the density of the electric charge on the patient's skin surface worsens the functioning conditions of the system of the acupuncture points and meridians, which in turn impedes a recovery of the organism.

It is the object of the invention to increase the curing influence due to a reduction of the leakage of the free electric charge from the skin surface of the patient's body.

The specific result is achieved by the fact that in a device for polyfactorial curing which includes two layers of woven, knitted or needle-punched fabric and the like, between which at least one screen with a high reflection factor for the electromagnetic waves of the ultrahigh frequency (UHF) and the infra-red spectrum is arranged, which is made of a synthetic film having a metallization layer, the screen according to the invention additionally includes a layer of a polymeric material which is applied to the metallization layer.

The device for polyfactorial curing can be made as a blanket, a bandage, a scarf, as an element of clothes or of a rug.

The invention is explained by a drawing in which a sectional view of the device, which is made, for example, as a blanket, is schematically represented.

The blanket 1 includes two layers 2, 3, made of a woven, knitted or needle-punched fabric, and a screen 4 with a high reflection factor for the electromagnetic waves of the ultrahigh frequency (UHF) and the infra-red spectrum. The screen 4 consists of a synthetic film 5 with a metallization layer, on the top which a layer 7 of a polymeric material is applied.

The device is intended for a polyfactorial curing of patients. It can be used in hospitals, in out-patient departments or for domiciliary treatment. It is applied as an independent remedy and also in combination with traditional kinds of treatment. It can be used for preventing and curing the following diseases:
- diseases of the peripheral nervous system (neuralgia and neuritis of the trigeminal nerve, neuritis of the facial nerve, neuralgia of the occipital nerves, thoracocervical radiculitis, intercostal neuralgia, lumbosacral radiculitis, polyradiculoneuritis, polyneuritis, traumata of the peripheral nerves);
- diseases of the vegetative nervous system (ganglionitis of the sympathetic trunk, plexitis solaris, hypothalamus syndrome, migraine, Raynaud's disease, diseases by concussions);
- diseases of the brain (cerebral atheriosclerosis, infectious diseases of the brain, brain traumata);
- diseases of the respiratory organs (systemic respiratory disease, systemic respiratory viral infection, tracheitis, bronchitis, bronchial asthma, bronchiectasis, acute and chronic pneumonia, pulmonary abscess, pleurisy);
- diseases of the digestive organs (gastritis, chronic gastritis, ulcer of the stomach and of the duodenum, dysfunction of the stomach and of the intestine);
- neurosis (neurasthenia, hysteria);
- metabolic disturbances and involutional changes (diabetes, climacteric syndrome);
- diseases of the cardiovascular system (ischaemic disease of the heart, hypertonic disease, obliteration diseases of the peripheral vessels, diseases of the veins);
- diseases of the supporting apparatus and the motor system (arthritis, arthrosis, osteochondrosis, myositis, neuromyositis of various aetiology, traumata).

The course of the curing lasts 15 to 20 days (1 to 3 procedures per day). The time of exposition is defined individually: depending on the feeling of the patient or on the parameters of the adaptation reactions. In the first case, the duration of the curing procedure is choosen from an interval of 12 to 40 minutes. In the second case, the duration of the procedures is corrected to one of the three time ranges: 12 to 22, 22 to 43, 46 to 70 minutes.

In most of the cases, the patient is completely enveloped in the blanket, but it is also possible to envelop a part of the body or to place the blanket on the required surface (as segments).

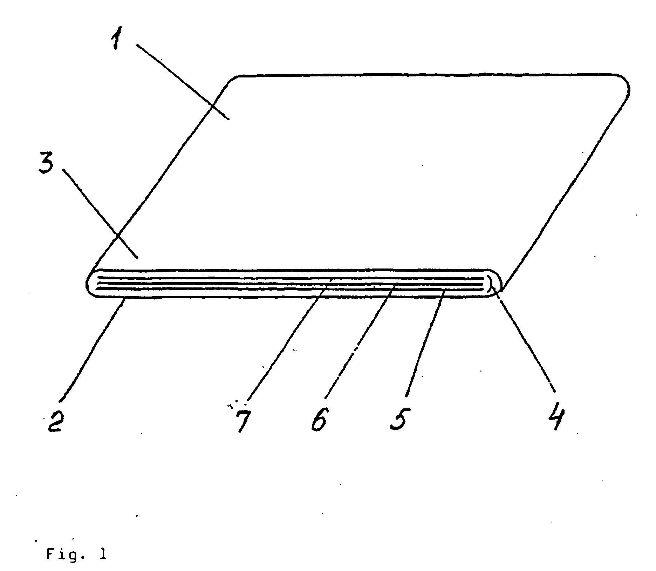

The patient (not shown in the drawing) wearing linen which contains a minimum of synthetic additives (possibly without clothes) is enveloped up in the blanket 1 and left to sit or lie in a convenient position for the time of the curing procedure. The patient should relax, if possible. The screen 4 protects the patient from external electrostatic and electromagnetic fields. The own electromagnetic radiation of the patient in the UHF range and the infra-red spectrum is reflected from the metallization layer 6 and exerts its influence on the patient's body. As the radiated frequencies in the UHF range correspond to the character of the occuring disturbances in the biological cells, the effect by this radiation on the biological cells results in the restoration of a correct functioning of the cells, there is a normalization of the control systems by the recovery processes and the the cellular homoiostasis is maintained.

The effect on the patient by his own radiation of the infra-red spectrum results in a decrease of the heat emission, an increase of the skin temperature which in turn promotes a change of the regional blood flow, of the general heat exchange and renders a beneficial effect on the functional condition of the regional and the central haemodynamics.

Prior to performing the procedure by the electrostatic field of the Earth, the patient was exposed to a negative electric charge, which was distributed on his skin surface non-uniformly. Upon enveloping the patient with the blanket 1, the metallization layer 6, which is isolated from the patient, creates a local milieu in which no external electrostatic fields have influence. Due to the absence of fields, the free electric charge is uniformly distributed on the skin surface of the patient. It creates favourable conditions for the functioning of the system of acupuncture points and meridians, which leads to an increase of the efficiency of said system for an adjustment of the energetic equilibrium of the organism. A leakage of the free charge from the skin surface towards the metallization layer 6 occurs through the layer 3 made of woven, knitted or needle-punched fabric and through the layer 7 of polymeric material. Consequently, the resistance to the leakage current is the sum of the resistance of the cloth 3 and the polymeric material 7. The resistance of the polymeric material 7 exceeds by several degrees the resistance of the cloth 3 and, consequently, the leakage current of the free electric charge from the skin surface of the patient will decrease by several degrees.

By the achievement of the specified technical results, the object of an increase of the efficiency of a polyfactorial curing is solved. The device can be used for preventing and curing diseases of the central nervous system, of the vegetative nervous system, of the respiratory organs, of the digestive organs, metabolic disturbances and involutional changes, diseases of the cardiovascular system, of the supporting apparatus and the motor system.

## Claims

1. A device for polyfactorial curing, including two layers of woven, knitted or needle-punched fabric, between which at least one screen with a high reflection factor for the electromagnetic waves of the ultrahigh frequency (UHF) and the infra-red spectrum is arranged, which is made of a synthetic film having a metallization layer, **characterized in that**
the screen additionally includes a layer of a polymeric material which is applied to the metallization layer.

2. The device according to claim 1, **characterized in that** it is made as a blanket, a bandage, a scarf, an element of clothing or of a rug.
